Europäisches Patentamt

European Patent Office

Office européen des brevets

(19)

(11) Numéro de publication : **0 091 895**
**B1**

# FASCICULE DE BREVET EUROPÉEN

(12)

(45) Date de publication du fascicule du brevet :
**28.05.86**

(21) Numéro de dépôt : **83870040.9**

(22) Date de dépôt : **06.04.83**

(51) Int. Cl.⁴ : **A 61 M 25/00**

(54) **Dispositif de drainage suprapubique de la vessie, introduit par l'urètre.**

(30) Priorité : **14.04.82 BE 207811**
**28.06.82 BE 208473**

(43) Date de publication de la demande :
**19.10.83 Bulletin 83/42**

(45) Mention de la délivrance du brevet :
**28.05.86 Bulletin 86/22**

(84) Etats contractants désignés :
**AT CH DE FR GB IT LI LU NL SE**

(56) Documents cités :
**FR-A- 2 161 950**
**US-A- 3 651 807**
**US-A- 3 920 023**
**US-A- 4 239 042**

(73) Titulaire : **Wildemeersch, Dirk Alfons August**
**Pierslaan, 125**
**B-8300 Knokke-Heist (BE)**

(72) Inventeur : **Wildemeersch, Dirk Alfons August**
**Pierslaan, 125**
**B-8300 Knokke-Heist (BE)**

(74) Mandataire : **Prignot, Jean et al**
**OFFICE HANSSENS SPRL Square Marie-Louise, 40 -**
**bte 19**
**B-1040 Bruxelles (BE)**

## Description

La présente invention se rapporte à un dispositif de drainage suprapubique de la vessie, destiné à être introduit par l'urètre.

Les dispositifs de drainage suprapubiques de la vessie sont connus de longue date, notamment en chirurgie de l'homme.

Au cours de ces dernières années, s'est développée en chirurgie de la femme une technique de drainage suprapubique de la vessie, mettant en œuvre notamment des cathéters de section réduite.

L'intérêt d'un dispositif de drainage suprapubique de la vessie, par rapport au dispositif de drainage par l'urètre utilisé de longue date en chirurgie de la femme (cathéter de Foley) a été établi par de nombreuses études et communications publiées depuis les années 1960 à nos jours.

Les avantages reconnus de pareils dispositifs sont notamment :

confort accru pour la patiente ;

réduction importante des causes d'infection ;

réduction importante des soins hospitaliers au cours de la période de drainage de la vessie ;

possibilité pour la patiente de commencer une miction normale alors même que le cathéter est en place.

Toutefois, la plupart des dispositifs de drainage suprapubiques de la vessie connus à ce jour sont prévus pour être introduits dans la vessie à partir de la paroi abdominale. Pareil mode opératoire nécessite l'intervention d'un spécialiste, afin d'introduire de manière sûre le cathéter dans la vessie sans causer de dommages aux organes voisins.

La présente invention a pour objet un dispositif de drainage suprapubique de la vessie du type pouvant être introduit par l'urètre. Ce mode opératoire rend le placement du cathéter beaucoup plus simple puisqu'il s'effectue au départ de la vessie en direction de la paroi abdominale en contournant la symphyse. De la sorte, le cathéter aboutit de manière certaine dans la vessie sans que celle-ci ne risque d'être percée de part en part, et le positionnement correct du point de percée de la paroi abdominale est obtenu beaucoup plus aisément à partir de la vessie. Un tel dispositif ne nécessite de ce fait plus l'intervention obligatoire d'un spécialiste pour le placement du cathéter.

Un dispositif de ce type a déjà été décrit au brevet US-A-3.920.023. Ce document révèle en effet un dispositif suprapubique de drainage de la vessie comportant une pointe acérée attachée à un cathéter destiné à constituer drain, ainsi qu'une sonde de longueur suffisante pour traverser l'urètre et s'étendre jusqu'à la paroi de la vessie. Le dispositif comporte également un piston, destiné à recevoir l'extrémité du cathéter opposée à l'aiguille et à coulisser dans la sonde.

Ce dispositif présente toutefois divers inconvénients. En effet, il n'est pas conçu pour permettre un remplissage préalable de la vessie, et nécessite donc une courbure importante de la sonde pour parvenir à la paroi abdominale, ce qui peut rendre le passage de l'urètre relativement malaisé.

En outre, le cathéter utilisé, tout en étant souple, doit cependant être de rigidité suffisante pour permettre la transmission de la force exercée par le piston à la pointe acérée.

Enfin, la longueur de la sonde, ainsi que la présence d'un piston pourvu d'un logement pour recevoir l'extrémité du cathéter, augmentent le coût de production du dispositif, et rendent son utilisation compliquée.

D'autre part, la publication FR-A-2.161.950 enseigne l'usage, pour le placement d'un drain, d'une aiguille chirurgicale solidaire d'un drain, aiguille pourvue dans sa région de préhension de parties plates opposées facilitant sa manipulation. Le dispositif décrit dans ce brevet n'est toutefois pas adapté au placement d'un drain suprapubique dans la vessie à partir de l'urètre. En effet, en premier lieu il ne comprend pas de moyens pour amener sans dommages l'aiguille courbée au travers de l'urètre jusque dans la vessie. En outre, la forme de l'aiguille, la surface réduite de l'élément de manipulation et l'absence de repères sur le drain constituent autant d'éléments qui rendent l'usage de ce dispositif peu approprié à son utilisation en tant qu'élément d'un dispositif de placement d'un drain suprapubique dans la vessie à partir de l'urètre.

L'invention a dès lors pour objet de fournir un dispositif de drainage suprapubique de la vessie introduit par l'urètre qui remédie aux divers inconvénients des dispositifs précités.

Ce but est atteint en prévoyant un dispositif suprapubique de drainage de la vessie introduit par l'urètre, comportant une pointe acérée attachée à un cathéter destiné à constituer drain et une sonde passant dans l'urètre pour permettre le passage de la pointe acérée et du cathéter dans la vessie, dans lequel la pointe acérée est constituée par la partie avant d'une aiguille incurvée, comportant des moyens de solidarisation avec le cathéter et de longueur supérieure à celle de la sonde, et dans lequel la sonde, destinée à être introduite seulement dans l'urètre, est agencée pour permettre le passage de l'aiguille incurvée.

Suivant une caractéristique supplémentaire de l'invention, l'aiguille comporte deux zones, à savoir une zone avant, constituant partie active de l'aiguille, se terminant à l'avant en la pointe acérée et se raccordant à l'arrière à une zone arrière constituant organe de préhension et de manipulation de l'aiguille, au moins la zone avant étant au moins partiellement de forme incurvée, la courbure étant telle que la pointe se dispose suivant un angle compris entre 115 et 145° par rapport à l'arrière de la partie active, l'aiguille comportant des moyens de solidarisation avec le cathéter.

Suivant une caractéristique supplémentaire de l'invention, la zone avant de l'aiguille est constituée d'une partie de tête rectiligne, dans laquelle est formée la pointe, prolongée par une partie en arc de cercle d'angle au centre de 35 à 55° et de rayon de courbure compris entre 6 et 10 cm, cette partie en arc de cercle se raccordant à une partie rectiligne constituant l'arrière de la partie active, qui se prolonge pour former l'organe de préhension et de manipulation.

Suivant encore une caractéristique supplémentaire de l'invention, il est formé dans la zone arrière constituant organe de préhension et de manipulation de l'aiguille, au moins un logement pour recevoir un ergot formé à la face inférieure d'un poucier, en vue de permettre une solidarisation momentanée du dit poucier et de l'aiguille.

Suivant encore une autre caractéristique supplémentaire de l'invention, la zone arrière de l'aiguille comporte au moins deux perforations diamétrales alignées, destinées à coopérer avec deux ergots formés à la face inférieure du poucier, ces ergots étant formés dans la partie médiane d'une rainure semi-cylindrique épousant la forme du corps de l'aiguille.

Suivant une autre caractéristique supplémentaire de l'invention, la zone avant et la zone arrière de l'aiguille sont incurvées.

Suivant encore une autre caractéristique supplémentaire de l'invention, les moyens de solidarisation de l'aiguille avec le cathéter sont constitués par un élément de solidarisation agencé à l'extrémité de la zone arrière de l'aiguille.

Suivant une caractéristique supplémentaire de l'invention, l'élément de solidarisation à l'extrémité de la zone arrière de l'aiguille, est un des éléments d'un dispositif de solidarisation à pas de vis, et les dimensions externes de ce dispositif de solidarisation, en section transversale, sont au maximum égales aux dimensions externes de l'aiguille en section transversale.

Suivant une autre caractéristique supplémentaire de l'invention, l'élément de solidarisation à l'extrémité de la zone arrière de l'aiguille est un téton coaxial à l'aiguille, de diamètre correspondant au diamètre interne du cathéter, destiné à être introduit dans l'extrémité avant dudit cathéter et à être solidarisé de cette dernière par collage.

Suivant encore une autre caractéristique supplémentaire de l'invention, les moyens de solidarisation de l'aiguille avec le cathéter sont constitués par un canal longitudinal formé dans l'aiguille, permettant le passage du cathéter.

Suivant une autre caractéristique supplémentaire de l'invention, le cathéter destiné à constituer drain suprapubique de la vessie présente à une extrémité un élément de solidarisation complémentaire de celui prévu à l'extrémité de l'aiguille, et à l'autre extrémité une partie perforée constituant dispositif de drainage, les dimensions externes, en section transversale du cathéter, étant au maximum égales aux dimensions externes, en section transversale de l'aiguille.

Suivant une caractéristique supplémentaire de l'invention, le cathéter comporte, à sa surface externe, des repères donnant une indication de la distance séparant le point considéré de l'extrémité de drainage.

Suivant encore une autre caractéristique supplémentaire de l'invention, la sonde pour l'introduction par l'urètre du dispositif de drainage suprapubique de la vessie comporte un corps tubulaire constituant canal, de dimensions internes, en section transversale, au moins légèrement supérieures aux dimensions externes, en section transversale, de l'aiguille, des moyens étant prévus pour permettre le passage de l'aiguille incurvée.

Suivant une caractéristique supplémentaire de l'invention, le corps de la sonde est de souplesse suffisante pour permettre par sa déformation le passage de l'aiguille.

Suivant encore une caractéristique supplémentaire de l'invention, le corps est rectiligne et sa souplesse est renforcée par la présence d'une fente longitudinale s'étendant, depuis l'avant du corps, sur une distance comprise entre la moitié et la totalité de la longueur du corps.

Suivant une autre caractéristique supplémentaire de l'invention, le corps de la sonde présente une courbure correspondant sensiblement à celle de la partie la plus incurvée de l'aiguille, et sa souplesse est suffisante pour permettre le passage des autres parties de l'aiguille.

Suivant encore une autre caractéristique supplémentaire de l'invention, le corps de la sonde est en une matière rigide et affecte une forme générale tronconique, de diamètre croissant depuis sa partie antérieure jusqu'à sa partie postérieure, et le canal tronconique qu'il forme est de dimensions suffisantes pour permettre le passage de l'aiguille incurvée.

Suivant encore une caractéristique supplémentaire de l'invention, le corps de la sonde est suivi d'une embouchure allant en s'évasant depuis le corps jusqu'à son extrémité postérieure, ladite embouchure comportant des moyens de raccordement à une seringue.

Suivant une autre caractéristique supplémentaire de l'invention, la sonde présente un organe de préhension, à la partie postérieure du corps, de forme générale plane, s'étendant transversalement au corps et constituant organe de retenue de la sonde en position active.

Suivant une caractéristique supplémentaire de l'invention, l'organe de préhension est amovible et se présente sous la forme d'une plaquette percée d'un orifice de dimension correspondant à la section transversale du corps, cette plaquette étant engagée sur le corps jusqu'à venir buter contre l'embouchure évasée de la sonde.

Ces buts et d'autres encore seront mieux compris en se reportant à la description ci-après, en même temps qu'au dessin annexé, qui représente uniquement à titre d'exemple, divers modes de réalisation de l'invention et dans lequel :

la figure 1 représente un dispositif suivant l'invention, en cours d'introduction par l'urètre,

la figure 2 est une vue en élévation de l'aiguille d'un dispositif suivant l'invention,

la figure 3 est une vue en perspective d'un autre mode de réalisation de l'aiguille, solidarisée d'un poucier,

la figure 4 est une vue en élévation d'encore un autre mode de réalisation de l'aiguille,

la figure 5 représente, à plus grande échelle, un dispositif de solidarisation entre l'aiguille et le cathéter,

la figure 6 est une vue d'un autre mode de réalisation d'un dispositif de solidarisation de l'aiguille et du cathéter,

la figure 7 montre un corps de sonde en matière souple, se déformant au passage d'une partie incurvée de l'aiguille,

la figure 8 montre une sonde courbe et son embouchure, au passage d'une partie incurvée de l'aiguille,

la figure 9 représente, en perspective, un corps de sonde rectiligne, pourvu d'une fente longitudinale, dans lequel est engagée une partie incurvée de l'aiguille,

la figure 10 est une vue en élévation d'une sonde en matière rigide, présentant un corps tronconique,

la figure 11 montre, de face, une plaquette constituant organe de préhension de la sonde,

la figure 12 est une vue en élévation, et partiellement en coupe, d'un autre mode de réalisation de l'aiguille et du cathéter d'un dispositif suivant l'invention.

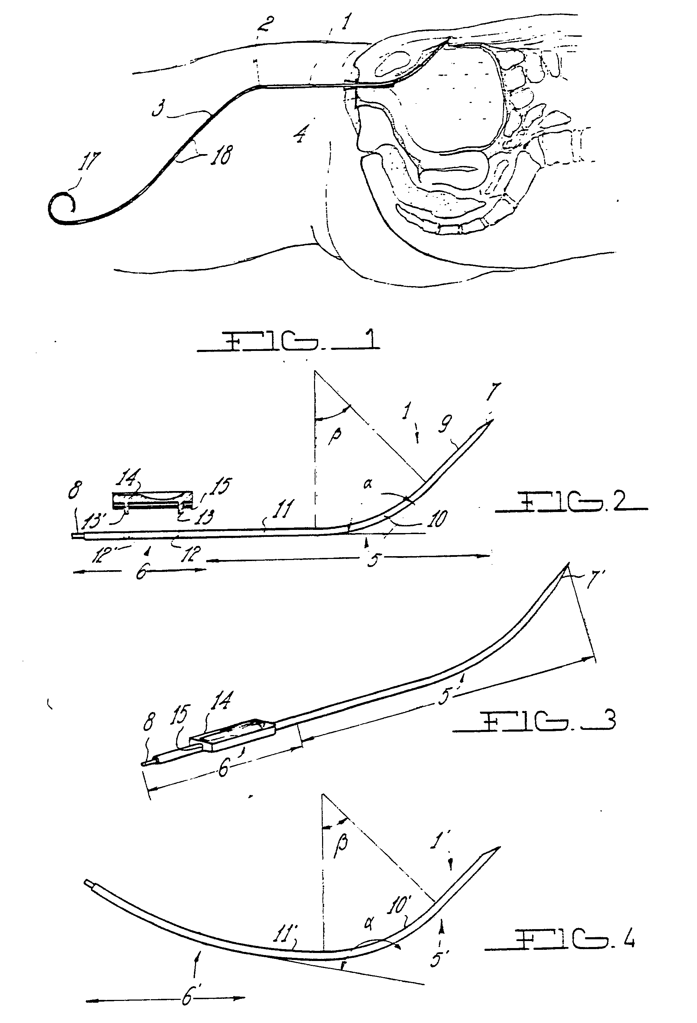

Afin de mieux faire comprendre le maniement du dispositif de l'invention, la figure 1 représente un tel dispositif en cours d'introduction par l'urètre. Ce dispositif comprend essentiellement : une aiguille 1, raccordée par un dispositif de solidarisation 2 à un cathéter 3 constituant le drain proprement dit, et une sonde 4. L'aiguille 1 incurvée est destinée à assurer l'entraînement du cathéter 3 au travers. de l'urètre, à assurer le percement de la paroi de la vessie et de la paroi abdominale, et à entraîner l'extrémité du cathéter 3, dont elle est solidaire par l'intermédiaire du dispositif de solidarisation 2, au travers de la percée réalisée dans la paroi de la vessie et la paroi abdominale jusqu'à ce que l'extrémité postérieure du cathéter 3 se dispose convenablement dans la vessie afin d'en assurer le drainage.

La sonde 4 a pour but de permettre le passage de l'aiguille incurvée au travers de l'urètre. Toutefois, afin de permettre un placement correct du drain, la vessie est au préalable remplie d'une solution d'eau physiologique, et dans ce but, la sonde 4 est avantageusement pourvue de moyens permettant son raccordement à une seringue.

Les caractéristiques de chacun des éléments utilisés apparaîtront plus clairement de la description détaillée donnée ci-après, en se reportant aux figures 2 à 12.

Les figures 2 à 4 et 12 représentent divers modes de réalisation de l'aiguille 1 d'un dispositif suivant l'invention.

Dans chacun de ces modes de réalisation, l'aiguille, dont la longueur est comprise entre 17 et 23 cm peut être divisée en une zone avant, constituant partie active 5 de l'aiguille, qui s'étend sur environ les 2/3 de l'aiguille, et une zone arrière, constituant organe de préhension et de manipulation 6 de l'aiguille.

Dans la présente description, on entend par partie active 5 de l'aiguille la partie de l'aiguille qui, dans la position représentée en figure 1, s'étend depuis la paroi abdominale au travers de la vessie et de l'urètre jusqu'à la sortie de la sonde 4.

La zone avant ou partie active 5 de l'aiguille se termine à l'avant par une pointe acérée 7 ; la partie active est en outre incurvée de telle sorte que la pointe 7 se dispose suivant un angle $\alpha$ compris entre 115° et 145° par rapport à l'arrière de la partie active.

Dans les modes de réalisation représentés aux figures 2 à 4, la zone arrière 6 se termine en un élément de solidarisation 8.

En se reportant plus particulièrement aux modes de réalisation des figures 2 et 3, la zone avant 5 est constituée d'une partie de tête 9 rectiligne, dans laquelle est formée la pointe, prolongée par une partie en arc de cercle 10 d'angle au centre $\beta$ de 35 à 65° et de rayon de courbure compris entre 6 et 10 cm, cette partie en arc de cercle se raccordant à une partie rectiligne constituant l'arrière 11 de la partie active. Cette partie rectiligne 11 se prolonge en l'organe de préhension et de manipulation 6 de l'aiguille.

Suivant un mode de réalisation préféré de l'invention, il est formé dans la zone arrière de l'aiguille constituant organe de préhension et de manipulation 6 au moins un logement 12 destiné à recevoir un ergot 13 formé à la face inférieure d'un poucier 14, afin de permettre une solidarisation momentanée dudit poucier 14 et de l'aiguille 1.

De la sorte, une prise suffisante est assurée sur l'aiguille 1 pour permettre qu'elle soit forcée au travers de la sonde 4 et pour qu'elle soit tenue de manière sûre au cours du percement de la vessie et de la paroi abdominale.

Suivant le mode de réalisation de la figure 2, il est formé dans la zone arrière 6 de l'aiguille deux logements 12, 12' en forme de perforations diamétrales alignées, destinées à coopérer avec deux ergots 13, 13' formés à la face inférieure du poucier 14. Ces ergots 13, 13' sont constitués dans la partie médiane d'une rainure semi-cylindrique 15 épousant la forme de l'aiguille.

Le mode de réalisation de la figure 3 est sensiblement identique à celui de la figure 2, sauf que le poucier 14 a été représenté solidarisé de l'aiguille, et que la pointe 7' de l'aiguille est une pointe conique, alors que la pointe 7 représentée en figure 2 est une pointe biseautée.

Suivant le mode de réalisation de la figure 4, la zone avant 5' et la zone arrière 6' de l'aiguille 1' sont incurvées, la partie 11' de la zone avant et la zone arrière 6' présentant toutefois un rayon de courbure supérieur à celui de la partie en arc de cercle 10', d'angle au centre $\beta$ de 35 à 65° à la zone avant.

La figure 5 montre à plus grande échelle un dispositif de solidarisation 2 entre l'aiguille 1 et le cathéter 3, constitué d'une vis 8 formée à l'extrémité arrière de l'aiguille 1 et un chambrage taraudé 8' constitué à l'avant du cathéter 3.

La figure 6 montre, également à plus grande échelle, un dispositif de solidarisation 2' entre une aiguille 1 et un cathéter 3, constitué d'un ergot 8'', coaxial à l'aiguille et de diamètre correspondant au diamètre interne du cathéter, introduit dans le conduit 16 du cathéter. La jonction de l'aiguille et du cathéter s'effectue par exemple par collage de l'ergot 8'' dans le conduit 16.

En tout état de cause, on veillera à ce que les dimensions externes en section transversale du cathéter 3 jusque et y compris à l'endroit du dispositif de solidarisation 2 à l'aiguille ne soient pas supérieures aux dimensions externes en section transversale de l'aiguille 1.

De préférence, on utilisera un cathéter 3 ayant, comme représenté en figure 1, une extrémité 17 préformée en boucle, reprenant sa forme après le passage de l'urètre. Dans de tels cathéters, les orifices de drainage sont formés dans la face interne de la boucle.

Suivant un mode de réalisation préféré de l'invention, des repères 18, de préférence annulaires, sont formés sur la paroi externe du cathéter, donnant des indications sur la distance séparant l'endroit considéré de l'extrémité de drainage du cathéter.

Les figures 7 à 10 représentent divers modes de réalisation d'une sonde 4 d'un dispositif suivant l'invention, destinée à permettre le passage de l'aiguille 1 et du cathéter 3 au travers de l'urètre.

Suivant le mode de réalisation de la figure 7, la sonde comporte essentiellement un corps 19, normalement rectiligne, mais suffisamment souple pour se déformer élastiquement de la manière représentée à la figure 7 au passage de la partie incurvée 10 de l'aiguille. Le diamètre interne du corps de sonde 19 est légèrement supérieur au diamètre externe de l'aiguille.

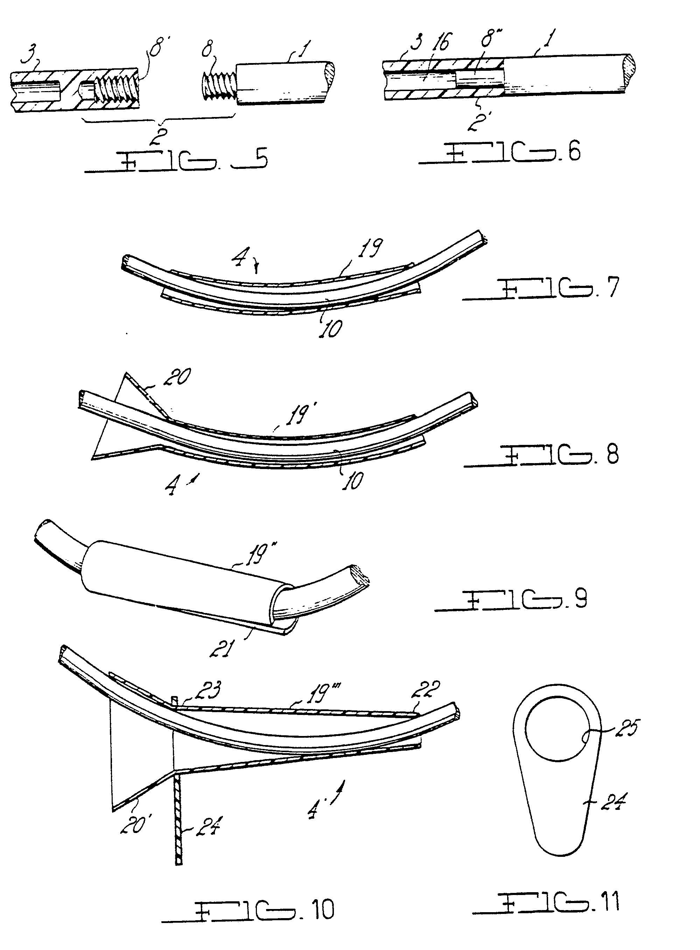

En se reportant au mode de réalisation de la figure 8, la sonde 4 est constituée d'un corps souple 19' incurvé, se raccordant à sa partie arrière à une embouchure 20. Suivant ce mode de réalisation, la courbure du corps 19' correspond sensiblement à la courbure de la partie incurvée 10 de l'aiguille. Dans ce cas également, la sonde est réalisée en une matière suffisamment souple pour se déformer de manière élastique au passage de l'aiguille, la déformation se produisant toutefois au passage de la partie rectiligne de l'aiguille.

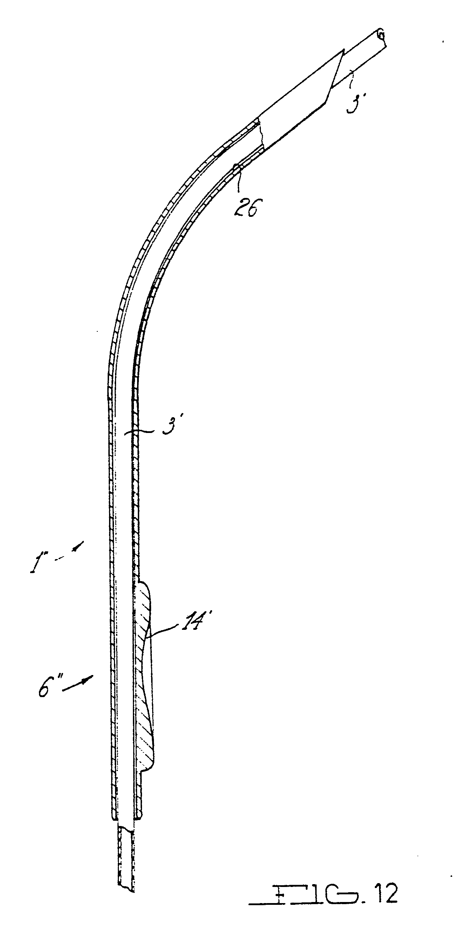

Suivant le mode de réalisation de la figure 9, la souplesse du corps rectiligne 19'' est obtenue, ou à tout le moins renforcée, par la présence d'une fente longitudinale 21, s'étendant depuis l'extrémité avant du corps, sur une distance comprise entre la moitié et la totalité de la longueur du corps. Au passage de la partie incurvée 10 de l'aiguille, l'ouverture de la fente 21 confère au corps 19'' la souplesse requise.

Enfin, suivant le mode de réalisation de la figure 10, une sonde 4' est réalisée en une matière rigide et comporte un corps 19'' tronconique, de diamètre croissant depuis sa partie antérieure 22 jusqu'à sa partie postérieure 23, les dimensions internes du corps étant suffisantes pour permettre le passage de la partie incurvée 10 de l'aiguille. Ce corps 19'' se raccorde à son extrémité postérieure à une embouchure 20' prévue pour permettre le raccordement à une seringue. Le corps 19'' est également solidarisé à son extrémité postérieure, à un organe de préhension 24 se présentant sous forme d'une plaquette. Suivant un mode de réalisation préféré de 1' invention, la plaquette est amovible et se présente par exemple comme la plaquette 24 représentée en figure 11. Celle-ci est de forme générale allongée, et présente un orifice 25 pour le passage du corps de la sonde ; elle vient buter contre l'embouchure 20' lorsqu'elle est insérée sur le corps 19'''.

La plaquette 24 constitue organe de retenue de la sonde 4 lorsque celle-ci est soumise à déformation ou à effort au passage de l'aiguille, et organe de préhension lorsque le dispositif de drainage a été mis en place et que la sonde peut être retirée de l'urètre.

Suivant le mode de réalisation de la figure 12, l'aiguille 1'' est creuse et comporte de ce fait un canal longitudinal 26 s'étendant sur toute la longueur de l'aiguille au travers duquel peut passer un cathéter 3'.

Cette aiguille présente également dans sa zone arrière 6'' constituant organe de préhension et de manipulation, un poucier 14' qui, dans l'exemple représenté, est constitué d'une pièce avec l'aiguille 1''.

En pratique, ce dispositif permet un placement extrêmement aisé et rapide d'un dispositif de drainage suprapubique de la vessie.

En effet, la sonde 4 est d'abord mise en place dans l'urètre. Elle est ensuite raccordée à une seringue de manière à remplir la vessie d'eau physiologique. On déconnecte ensuite la seringue et l'on introduit par la sonde 4 l'aiguille 1 solidaire du cathéter 3. L'aiguille 1 est forcée au travers de la sonde, alors que la plaquette 24 maintient cette dernière en place, même lorsqu'elle est soumise à des efforts en vue d'aboutir à une déformation autorisant le passage de l'aiguille. La forme incurvée de l'aiguille permet alors de contourner la symphyse et de percer la paroi de la vessie pour aboutir à la paroi abdominale.

Lorsqu'il est fait usage d'une aiguille telle que représentée aux figures 2 à 4, une fois les divers tissus traversés, l'aiguille 1 est désolidarisée du poucier 14, dans la mesure où celui-ci a été utilisé, et est retirée par l'orifice formé, en entraînant le cathéter 3. Les indications 18 permettent de déterminer la longueur du cathéter demeurant dans la vessie, et le cathéter est alors découpé à la longueur voulue et raccordé à un dispositif de collecte conventionnel comprenant par exemple un récipient souple hermétique et une vanne autorisant ou interrompant le passage de l'urine.

Par contre, lorsqu'il est fait usage d'une aiguille creuse telle que représentée en figure 12, une fois les divers tissus traversés, il est introduit dans l'aiguille un cathéter 3, qui est déplacé au travers de l'aiguille 1″ jusqu'à occuper la position convenable en vue du drainage de la vessie.

L'aiguille est alors retirée en veillant à maintenir le cathéter en place.

Ce retrait peut s'effectuer, comme lors de l'usage d'une aiguille suivant les modes de réalisation des figures 2 à 4, par l'orifice formé dans les tissus.

Il peut également s'effectuer par l'urètre, en faisant en sens inverse le trajet parcouru pour aboutir à la paroi abdominale. Dans pareil cas, la zone arrière 6″ demeure en dehors du corps de la patiente, et le poucier 14′ peut ainsi sans inconvénient être solidaire de l'aiguille 1″.

La conception extrêmement simple du dispositif de l'invention permet sa réalisation économique sous forme d'un ensemble jetable contenu dans un emballage stérile.

## Revendications

1. Dispositif suprapubique de drainage de la vessie introduit par l'urètre, comportant une pointe acérée (7) attachée à un cathéter (3) destiné à constituer drain et une sonde (4) passant dans l'urètre pour permettre le passage de la pointe acérée (7) et du cathéter dans la vessie, caractérisé en ce que la pointe acérée est constituée par la partie avant d'une aiguille incurvée (1), comportant des moyens de solidarisation (2) avec le cathéter (3) et de longueur supérieure à celle de la sonde (4), et en ce que la sonde (4), destinée à être introduite seulement dans l'urètre, est agencée pour permettre le passage de l'aiguille incurvée.

2. Dispositif suivant le revendication 1, caractérisé en ce que l'aiguille (1 ; 1′ ; 1″) comporte deux zones (5, 6 ; 5′, 6′ ; 6″), à savoir une zone avant (5 ; 5′), constituant partie active de l'aiguille, se terminant à l'avant en la pointe acérée (7 ; 7′) et se raccordant à l'arrière à une zone arrière (6 ; 6′ ; 6″) constituant organe de préhension et de manipulation de l'aiguille, au moins la zone avant (5 ; 5′) étant au moins partiellement de forme incurvée, la courbure étant telle que la pointe (7 ; 7′) se dispose suivant un angle compris entre 115 et 145° par rapport à l'arrière de la partie active, l'aiguille comportant des moyens de solidarisation (8) avec le cathéter.

3. Dispositif suivant la revendication 2, caractérisé en ce que la zone avant (5) de l'aiguille (1) est constituée d'une partie de tête rectiligne (9), dans laquelle est formée la pointe (7), prolongée par une partie en arc de cercle (10) d'angle au centre de 35 à 65° et de rayon de courbure compris entre 6 et 10 cm, cette partie en arc de cercle (10) se raccordant à une partie rectiligne constituant l'arrière (11) de la zone active et se prolongeant pour former l'organe de préhension et de manipulation (6).

4. Dispositif selon l'une quelconque des revendications 2 ou 3, caractérisé en ce qu'il est formé dans la zone arrière (6) de l'aiguille (1) constituant organe de préhension et de manipulation, au moins un logement (12) pour recevoir un ergot (13) formé à la face inférieure d'un poucier (14), en vue de permettre une solidarisation momentanée dudit poucier (14) et de l'aiguille (1).

5. Dispositif selon la revendication 4, caractérisé en ce que la zone arrière (6) de l'aiguille (1) comporte au moins deux perforations diamétrales (12, 12′) alignées, destinées à coopérer avec deux ergots (13, 13′) formés à la face inférieure du poucier (14), et en ce que ces ergots sont formés dans la partie médiane d'une rainure semi-cylindrique (15) épousant la forme du corps de l'aiguille.

6. Dispositif selon la revendication 2, caractérisé en ce que la zone avant (5′) et la zone arrière (6′) de l'aiguille (1′) sont incurvées.

7. Dispositif selon l'une quelconque des revendications 2 à 6, caractérisé en ce que les moyens de solidarisation de l'aiguille (1) avec le cathéter (3) sont constitués par un élément de solidarisation (8) agencé à l'extrémité de la zone arrière (6) de l'aiguille (1).

8. Dispositif selon la revendication 7, caractérisé en ce que l'élément de solidarisation (8) à l'extrémité de la zone arrière (6) de l'aiguille (1) est un des éléments d'un dispositif de solidarisation (2) à pas de vis, et en ce que les dimensions externes du dispositif de solidarisation (2), en section transversale, sont au maximum égales aux dimensions externes, en section transversale, de l'aiguille (1).

9. Dispositif selon la revendication 7, caractérisé en ce que l'élément de solidarisation à l'extrémité de la zone arrière (6) de l'aiguille (1) est un téton (8″) coaxial à l'aiguille, de diamètre correspondant au diamètre interne du cathéter (3), destiné à être introduit dans l'extrémité avant dudit cathéter et à être solidarisé de cette dernière par collage.

10. Dispositif selon l'une quelconque des revendications 2 à 6, caractérisé en ce que les moyens de solidarisation avec le cathéter sont constitués par un canal longitudinal (26) formé dans l'aiguille (1″), permettant le passage du cathéter (3′).

11. Dispositif suivant les revendications 8 ou 9, caractérisé en ce que le cathéter (3) présente, à son extrémité avant, un élément de solidarisation (8′, 16) complémentaire de celui (8 ; 8″) porté par l'extrémité de l'aiguille (1), les dimensions externes du cathéter (3), en section transversale, étant au maximum égales aux dimensions externes, en section transversale, de l'aiguille (1).

12. Dispositif suivant la revendication 11, caractérisé en ce que le cathéter (3) comporte, à sa surface externe, des repères (18) donnant une indication de la distance séparant le point considéré de l'extrémité de drainage du cathéter.

13. Dispositif suivant la revendication 1, caractérisé en ce que la sonde (4) comporte un corps (19 ; 19′ ; 19″ ; 19‴) tubulaire constituant canal, de dimension interne en section transversale au moins légèrement supérieure à la dimension externe en section transversale de l'aiguille (1), comportant des moyens (21) pour permettre le passage de l'aiguille incurvée.

14. Dispositif suivant la revendication 13, caractérisé en ce que le corps (19 ; 19' ; 19") de la sonde (4) est de souplesse suffisante pour permettre par sa déformation le passage de l'aiguille (1).

15. Dispositif suivant l'une quelconque des revendications 13 ou 14, caractérisé en ce que le corps (19") de la sonde (4) est rectiligne et en ce que sa souplesse est renforcée par la présence d'une fente longitudinale (21) s'étendant, depuis l'avant du corps (19"), sur une distance comprise entre la moitié et la totalité du corps.

16. Dispositif suivant l'une quelconque des revendications 13 ou 14, caractérisé en ce que le corps (19') de la sonde (4) présente une courbure correspondant sensiblement à celle de la partie incurvée (10) de l'aiguille (1), et en ce que sa souplesse est suffisante pour permettre le passage de la partie rectiligne de l'aiguille.

17. Dispositif suivant la revendication 13, caractérisé en ce que le corps (19''') de la sonde (4') est en une matière rigide, et affecte une forme générale tronconique de diamètre croissant de sa partie antérieure (22) vers sa partie postérieure (23), et en ce que le canal tronconique qu'il forme est de dimensions suffisantes pour permettre le passage de l'aiguille incurvée.

18. Dispositif suivant l'une quelconque des revendications 13 à 17, caractérisé en ce que le corps (19' ; 19''') de la sonde (4 ; 4') est suivi d'une embouchure (20 ; 20') allant en s'évasant depuis le corps jusqu'à son extrémité postérieure, ladite embouchure (20 ; 20') comportant des moyens de raccordement à une seringue.

19. Dispositif suivant l'une quelconque des revendications 13 à 18, caractérisé en ce que la sonde (4') comporte un organe de préhension (24), à la partie postérieure du corps (19'''), de forme générale plane, s'étendant transversalement au corps (19''') et constituant organe de retenue de la sonde en position active.

20. Dispositif suivant la revendication 19, caractérisé en ce que l'organe de préhension (24) est amovible et se présente sous forme d'une plaquette percée d'un orifice (25) de dimension correspondant à la section transversale du corps (19'''), cette plaquette étant engagée sur le corps jusqu'à venir buter contre l'embouchure évasée (20') de la sonde (4').

## Claims

1. Device for suprapubic drainage of the bladder introduced through the urethra, which has a sharp tip (7) attached to a catheter (3) designed to serve as drain and a sleeve (4) adapted to be introduced into the urethra to allow passing through of the sharp tip (7) and the catheter into the bladder, characterized in that the sharp tip is the fore part of a curved needle (1) equipped with means (2) for connecting said needle to the catheter (3), said needle being longer than the sleeve, and in that the sleeve (4), intended to be introduced only into the urethra, is adapted to allow passage for the curved needle.

2. Device according to claim 1, characterized in that the needle (1 ; 1' ; 1") consists of two portions, (5,6 ; 5', 6' ; 6"), i.e. a front portion (5 ; 5') constituting the active part of the needle, terminating at the front end in the sharp tip (7 ; 7') and joined at the rear to a back portion (6 ; 6' ; 6") constituting the gripping and manipulating element of the needle, at least the front portion (5 ; 5') being at least partly curved, the curvature being such that the tip (7 ; 7') forms an angle of between 115 and 145° with the rear of the active part, the needle being equipped with means (8) for connecting it to the catheter.

3. Device according to claim 2, characterized in that the front portion (5) of the needle (1) consists of a straight head portion (9) from which the tip (7) is formed, extended by a part in the form of an arc of a circle (10) with an angle at the center of 35° to 65° and a radius of curvature of 6 to 10 cm, this part in the form of an arc of a circle (10) being connected to a straight part constituting the rear (11) of the active part, which extends to form the gripping and manipulating element (6).

4. Device according to anyone of the claims 2 or 3, characterized in that, in the back portion (6) making up the gripping and manipulating element of the needle (1) there is at least one seat (12) designed to accommodate a pin (13) formed on the bottom surface of a thumb piece (14), to furnish a temporary connection between said thumb piece (14) and the needle (1).

5. Device according to claim 4, characterized in that, extending into the back portion (6) of the needle (1), are at least two diametrically aligned holes (12, 12') designed to accommodate two pins (13, 13') formed on the bottom surface of the thumb piece (14), these pins being formed in the median part of a semicylindrical groove (15) conforming to the shape of the body of the needle.

6. Device according to claim 2, characterized in that the front portion (5') and the back portion (6') of the needle (1') are curved.

7. Device according to anyone of the claims 2 to 6, characterized in that the means for connecting the needle (1) to the catheter (3) consist of an interlocking device (8) located at the end of the back portion (6) of the needle (1).

8. Device according to claim 7, characterized in that the interlocking device (8) at the end of the back portion (6) of the needle (1) is one of the elements of a screw-threaded interlocking device (2), and in that the outer dimensions of this interlocking device (2), in cross-section, to the maximum equal the outer dimensions of the needle (1) in cross-section.

9. Device according to claim 7, characterized in that the interlocking device at the end of the back portion (6) of the needle (1) is a stud (8") coaxial with the needle, whose diameter matches the inner diameter of the catheter (3), designed to be inserted into the front end of said catheter and joined to it by an adhesive.

10. Device according to anyone of the claims 2 to 6, characterized in that the means for connecting the needle to the catheter consist of a lengthwise canal (26) inside the needle (1″) through which the catheter (3′) is able to pass.

11. Device according to claims 8 or 9, characterized in that the catheter (3) has, at its fore end, an interlocking device (8′ ; 16) which fits together with that (8 ; 8″) provided at the end of the needle (1), the outer dimensions of the catheter (3) in cross-section being to the maximum equal to the outer dimensions of the needle (1) in cross-section.

12. Device according to claim 11, characterized in that the catheter (3) has reference marks (18) on its outer surface, which indicate the distance between that point and the drainage tip.

13. Device according to claim 1, characterized in that the sound or sleeve (4) posseses a tubular body (19 ; 19′ ; 19″ ; 19‴) constituting a canal, whose internal dimensions in cross-section are at least slightly larger than the external dimensions in cross-section of the needle (1), means (21) being provided to allow the passage of the curved needle.

14. Device according to claim 13, characterized in that the body (19 ; 19′ ; 19″) of the sound or sleeve (4) is of adequate flexibility so that it can be deformed to allow the needle (1) to pass through.

15. Device according to anyone of claims 13 or 14, characterized in that the body (19″) of the sound or sleeve (4) is straight and its flexibility is enhanced by the presence of a lengthwise slit (21) extending from the front of the sleeve body (19″) over a distance of between half and all of the length of the sleeve body.

16. Device according to anyone of claims 13 or 14, characterized in that the body (19′) of the sleeve (4) has a curvature which roughly matches that of the curved part (10) of the needle (1), and in that it is flexible enough so that the straight part of the needle can pass through it.

17. Device according to claim 13, characterized in that the body (19‴) of the sleeve (4′) is made of a stiff material and its shape is roughly that of a truncated cone whose diameter increases from its anterior part (22) to its posterior part (23), and in that the dimensions of the truncated-cone-shaped canal which it forms are sufficient to allow the curved needle to pass through.

18. Device according to anyone of the claims 13 to 17, characterized in that the body (19′ ; 19‴) of the sleeve (4 ; 4′) is followed by an opening (20 ; 20′) which widens from the body to its posterior end, said opening (20 ; 20′) being equipped with means for connection to a syringe.

19. Device according to anyone of claims 13 to 18, characterized in that the sleeve (4′) has a gripping element (24), roughly flat in shape, on the posterior part of the body (19‴), said element extending transversely to the body (19‴) and serving as the retaining device for keeping the sleeve in active position.

20. Device according to claim 19, characterized in that the gripping element (24) is detachable and is in the form of a plate machined with an opening (25) whose size matches the cross-section of the sleeve body (19‴), this plate being slid onto the sleeve body until it is stopped by the flared opening (20′) of the sleeve (4′).

## Patentansprüche

1. Suprapubische Vorrichtung zur Entwässerung der Harnblase, die durch die Harnröhre eingeführt wird, die eine Stahlspitze (7), welche an einem Katheter (3), der als Abzugsrohr vorgesehen ist, befestigt ist, und eine Sonde (4), die die Harnröhre durchsetzt, um den Durchgang der Stahlspitze (7) und des Katheters in die Harnblase zu erlauben, aufweist, dadurch gekennzeichnet, daß die Stahlspitze durch das vordere Teilstück einer gekrümmten Nabel (1) gebildet ist, die Verbindungsmittel (2) mit dem Katheter (3) und eine Länge, die größer als jene der Sonde (4) ist, aufweist, und dadurch, daß die Sonde (4), die bestimmt ist, nur in die Harnröhre eingeführt zu werden, ausgestattet ist, um den Durchgang der gekrümmten Nadel zu erlauben.

2. Vorrichtung nach Anspruch 1, dadurch gekennzeichnet, daß die Nadel (1 ; 1′ ; 1″) zwei Zonen (5, 6 ; 5′, 6′ ; 6″) aufweist, nämlich eine vordere Zone (5 ; 5′), die das aktive Teilstück der Nabel bildet, die nach vorne in der Stahlspitze (7 ; 7′) endet und sich hinten an eine hintere Zone (6 ; 6′ ; 6″) anschließt, die ein Greif- und Manipulationsorgan der Nadel bildet, wobei zumindest die vordere Zone (5 ; 5′) zumindest teilweise von gekrümmter Form ist, wobei die Krümmung eine solche ist, daß die Spitze (7 ; 7′) in einem Winkel eingeschlossen zwischen 115 und 145° in Bezug auf den hinteren Teil des aktiven Teilstücks angeordnet ist, wobei die Nadel Mittel für die Verbindung (8) mit dem Katheter aufweist.

3. Vorrichtung nach Anspruch 2, dadurch gekennzeichnet, daß die vordere Zone (5) der Nadel (1) aus einem geradlinigen Kopfteilstück (9), an welchem die Spitze (7) ausgebildet ist, gebildet ist, das sich über ein Teilstück als Kreisbogen (10) mit dem Zentriwinkel von 35 bis 65° und mit einem Krümmungsradius eingeschlossen zwischen 6 und 10 cm verlängert, wobei dieses Teilstück im Kreisbogen (10) sich an ein geradliniges Teilstück anschließt, das den hinteren Teil (11) der aktiven Zone bildet, und sich verlängert, um das Greif- und Manipulationsorgan (6) zu bilden.

4. Vorrichtung nach einem der Ansprüche 2 oder 3, dadurch gekennzeichnet, daß in der hinteren Zone (6) der Nadel (1), die das Greif- und Manipulationsorgan bildet, zumindest ein Sitz (12) für die Aufnahme eines Anschlages (13); der an der Unterseite eines Daumenringes (14) gebildet ist, ausgebildet ist, zum Zweck, um eine vorübergehende Verbindung besagten Daumenringes (14) und der Nabel (1) zu ermöglichen.

5. Vorrichtung nach Anspruch 4, dadurch gekennzeichnet, daß die hintere Zone (6) der Nadel (1) zumindest zwei diametral in einer Linie angeordnete Perforationen (12, 12') aufweist, die bestimmt sind, um mit zwei Anschlägen (13, 13'), die an der Unterseite des Daumenringes (14) ausgebildet sind, zusammenzuwirken, und dadurch, daß diese Anschläge an der mittleren Partie einer halbzylindrischen Rinne (15) ausgebildet sind, die die Form des Körpers der Nadel annimmt.

6. Vorrichtung nach Anspruch 2, dadurch gekennzeichnet, daß die vordere Zone (5') und die hintere Zone (6') der Nadel (1') gekrümmt sind.

7. Vorrichtung nach einem der Ansprüche 2 bis 6, dadurch gekennzeichnet, daß die Mittel für die Verbindung der Nabel (1) mit dem Katheter (3) aus einem Verbindungselement (8) gebildet sind, das am Ende der hinteren Zone (6) der Nadel (1) angeordnet ist.

8. Vorrichtung nach Anspruch 7, dadurch gekennzeichnet, daß das Verbindungselement (8) am Ende der hinteren Zone (6) der Nadel (1) eines der Elemente einer Einrichtung zur Verbindung (2) mit einem Gewindegang ist und dadurch, daß die äußeren Dimensionen der Verbindungseinrichtung (2), im Querschnitt, höchstens gleich den äußeren Dimensionen, im Querschnitt, der Nabel (1) sind.

9. Vorrichtung nach Anspruch 7, dadurch gekennzeichnet, daß das Verbindungselement am Ende der hinteren Zone (6) der Nadel (1) eine zur Nadel koaxiale Stichleitung (8") ist, von einem Durchmesser, der dem inneren Durchmesser des Katheters (3) entspricht, bestimmt, um in das vordere Ende besagten Katheters eingeführt zu werden und um mit diesem letzteren durch Verklebung verbunden zu werden.

10. Vorrichtung nach einem der Ansprüche 2 bis 6, dadurch gekennezeichnet, daß die Mittel für die Verbindung mit dem Katheter durch einem Längskanal (26) gebildet sind, der in der Nadel (1") hergestellt ist, der den Durchgang des Katheters (3) erlaubt.

11. Vorrichtung nach den Ansprüchen 8 oder 9, dadurch gekennzeichnet, daß der Katheter (3), an seinem vorderen Ende, ein Verbindungselement (8' ; 16) aufweist, das komplementär zu jenem (8, 8") ist, das vom Ende der Nadel (1) getragen wird, wobei die äußeren Dimensionen des Katheters (3), im Querschitt, höchstens gleich den äußeren Dimensionen, im Querschnitt, der Nadel (1) sind.

12. Vorrichtung nach Anspruch 11, dadurch gekennzeichnet, daß der Katheter (3) an seiner äußeren Oberfläche Sichtmarken (18) aufweist, die eine Angabe über die Distanz machen, die den in Betracht gezogen Punkt vom Ende des Abzugsrohrs des Katheters trennt.

13. Vorrichtung nach Anspruch 1, dadurch gekennzeichnet, daß die Sonde (4) einem rohrförmigen Körper (19 ; 19', 19", 19"') aufweist, der als Abzug bestimmt ist, von einer inneren Dimension im Querschnitt zumindest gering größer als die äußere Dimension im Querschnitt der Nadel (1), wobei sie Mittel (21) aufweist, um den Durchgng der gekrümmten Nadel zu erlauben.

14. Vorrichtung nach Anspruch 13, dadurch gekennzeichnet, daß der Körper (19 ; 19' ; 19") der Sonde (4) von einer ausreichenden Biegsamkeit ist, um durch seine Deformation den Durchgang der Nadel (1) zu erlauben.

15. Vorrichtung nach einem der Ansprüche 13 oder 14, dadurch gekennzeichnet, daß der Körper (19") der Sonde (4) geradlinig ist und dadurch, daß seine Biegsamkeit durch das Vorhandensein eines Längsschlitzes (21) erhöht wird, der sich ausgehend vom Vorderteil des Köpers (19") über eine Distanz, die zwischen der Hälfte und der ganzen Länge des Körpers enthalten ist, erstreckt.

16. Vorrichtung nach einem der Ansprüche 13 oder 14, dadurch gekennzeichnet, daß der Körper (19') der Sonde (4) eine Krümmung aufweist, die annähernd jener des gekrümmten Abschnitts (10) der Nadel (1) entspricht und dadurch, daß seine Schmiegsamkeit ausreichend ist, um den Durchgang des geradlinigen Abschnitts der Nadel zu erlauben.

17. Vorrichtung nach Anspruch 13, dadurch gekennzeichnet, daß der Körper (19"') der Sonde (4) aus einem steifen Material ist und eine im wesentlichen kegelstumpfartige Form annimmt, von einem Durchmesser, der von seinem vorderen Teilstück (22) zu seinem hinteren Teilstück (23) ansteigt, und dadurch, daß der Kegelstumpfartige Kanal, den er bildet, von Dimensionen ist, die ausreichend sind, um den Durchgang der gekrümmten Nadel zu erlauben.

18. Vorrichtung nach einem der Ansprüche 13 bis 17, dadurch gekennzeichnet, daß an den Körper (19' ; 19"') der Sonde (4 ; 4') ein Mundstück (20, 20') anschließt, das sich ausgehend vom Körper bis zu seinem hinteren Ende erweitert, wobei besagtes Mundstück (20, 20') Mittel für die Verbindung mit einer Injektionsspritze aufweist.

19. Vorrichtung nach einem der Ansprüche 13 bis 18, dadurch gekennzeichnet, daß die Sonde (4') ein Greiforgan (24) aufweist, am hinteren Teilstück des Körpers (19"') von einer im wesentlichen ebenen Form, das sich quer zum Körper (19"') erstreckt und ein Rückhaltorgan für die Sonde in der aktiven Position bildet.

20. Vorrichtung nach Anspruch 19, dadurch gekennzeichnet, daß das Greiforgan (24) abnehmbar ist und die Form einer Platte hat, die von einer Öffnung (25) von einer Größe, die dem Querschnitt des Körpers (19"') entspricht, durchsetzt ist, wobei diese Platte am Körper bis zur Anlage gegen das Mundstück (20'), das sich von der Sonde (4') ausweitet, eingesetzt ist.

0 091 895

FIG.1

FIG.2

FIG.3

FIG.4

FIG. 5

FIG. 6

FIG. 7

FIG. 8

FIG. 9

FIG. 10

FIG. 11

FIG. 12